(19) Europäisches Patentamt / European Patent Office / Office européen des brevets

(11) **EP 3 858 409 B1**

(12) **EUROPEAN PATENT SPECIFICATION**

(45) Date of publication and mention
of the grant of the patent:
**08.05.2024 Bulletin 2024/19**

(21) Application number: **19867937.5**

(22) Date of filing: **27.09.2019**

(51) International Patent Classification (IPC):
**A61M 16/00** *(2006.01)*    **A61B 5/087** *(2006.01)*
**A61M 16/10** *(2006.01)*    **A61B 5/00** *(2006.01)*
**A61B 5/08** *(2006.01)*    **A61M 16/16** *(2006.01)*
**B01D 53/047** *(2006.01)*

(52) Cooperative Patent Classification (CPC):
**A61M 16/101; A61B 5/0816; A61B 5/087;**
**A61B 5/7246; A61M 16/024; B01D 53/047;**
A61B 2505/07; A61M 16/0063; A61M 16/026;
A61M 16/0672; A61M 16/16; A61M 16/202;
A61M 2016/0021; A61M 2016/0027;
A61M 2016/0039;                    (Cont.)

(86) International application number:
**PCT/JP2019/038359**

(87) International publication number:
**WO 2020/067505 (02.04.2020 Gazette 2020/14)**

(54) **RESPIRATION RATE MEASUREMENT DEVICE**

VORRICHTUNG ZUR MESSUNG DER ATEMFREQUENZ

DISPOSITIF DE MESURE DE FRÉQUENCE RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.09.2018 JP 2018185785**

(43) Date of publication of application:
**04.08.2021 Bulletin 2021/31**

(73) Proprietor: **Teijin Pharma Limited**
**Tokyo 100-0013 (JP)**

(72) Inventors:
• **YAMAURA, Yuki**
**Tokyo 100-0013 (JP)**
• **MORISHITA, Yuta**
**Tokyo 100-0013 (JP)**
• **YOSHIZAWA, Akira**
**Tokyo 100-0013 (JP)**

(74) Representative: **Hoffmann Eitle**
**Patent- und Rechtsanwälte PartmbB**
**Arabellastraße 30**
**81925 München (DE)**

(56) References cited:
**JP-A- H06 190 045        JP-A- 2001 266 205
JP-A- 2002 102 187        JP-A- 2002 336 207
JP-A- 2014 068 782        JP-A- 2015 085 191
US-A1- 2015 230 759        US-A1- 2016 375 215**

**(Cont. next page)**

EP 3 858 409 B1

(52) Cooperative Patent Classification (CPC): (Cont.)
A61M 2202/0208; A61M 2205/3331;
A61M 2205/502; A61M 2230/42; B01D 2256/12;
B01D 2259/40007; B01D 2259/40009;
B01D 2259/4533

**Description**

FIELD

The present disclosure relates to a respiratory rate measurement device used for a pressure swing adsorption oxygen concentration device.

BACKGROUND

Oxygen therapy has been conventionally conducted as one medical treatment for patients suffering from respiratory diseases such as asthma and chronic obstructive pulmonary disease. In this therapy, the patients inhale oxygen gas or concentrated oxygen gas. In recent years, HOT (Home Oxygen Therapy) in which the patients inhale oxygen, e.g., at home or in facilities, aiming to improve the patients' QOL (Quality of Life), is becoming mainstream, and an oxygen concentration device is mainly used as an oxygen source.

The oxygen concentration device means a device that concentrates and delivers oxygen existing at about 21% in the air. Most oxygen concentration devices are generally of the pressure swing adsorption type (to be referred to as the PSA type hereinafter).

The PSA oxygen concentration device repeatedly performs an adsorption process of introducing air into an adsorption column filled with an adsorbent for selectively adsorbing nitrogen gas, and pressurizing the adsorption column to adsorb the nitrogen gas to the adsorbent, and a desorption process of depressurizing the adsorption column to exhaust the adsorbed nitrogen gas out of the system. Repeating the adsorption and desorption processes generates concentrated oxygen gas to allow the oxygen concentration device to continuously provide highly concentrated oxygen gas to the patient. A method for depressurizing the adsorption column to atmospheric pressure or less in the desorption process is sometimes called not PSA but, e.g., VPSA or VSA, but the basic principle of VPSA or VSA is the same as that of PSA, and these methods will therefore be collectively referred to as PSA hereinafter.

The major disease of patients who receive home oxygen therapy is chronic obstructive pulmonary disease (to be referred to as COPD hereinafter). COPD refers to an irreversible disease that produces symptoms of coughing and sputum expectoration or dyspnea on exertion, due to constriction of bronchi or destruction of pulmonary alveolar walls.

When the symptoms of COPD worsen, shortness of breath or an increase in respiratory rate is observed, and the clinical manifestations sometimes deteriorate until the occurrence of the "state in which a treatment change or any extra treatment may be preferably involved in a stable phase" called an acute exacerbation of COPD. When such acute aggravation of COPD occurs, the patients enter hospitals in most cases and may even lapse into respiratory failure or face crises of their lives. In addition, even when the patients are allowed to leave the hospitals, it is often the case that the patients develop severer symptoms in the stable phase than they were before their hospital stay, and are then repeatedly admitted to and discharged from hospitals.

In COPD, it is very important to detect signs or initial symptoms of an acute exacerbation in the patient as early as possible, and treat the patient early before his or her clinical manifestations worsen to the degree that he or she is hospitalized, and in home oxygen therapy, therefore, respiratory information of the patient, especially a change in respiratory rate of the patient, serves as a very beneficial source of information in terms of knowing the pathological condition of the patient.

The respiratory rate can be measured even using, e.g., a stomach belt, but to know a long-term change in pathological condition of the patient, the patient is forced to bear a burden as, e.g., he or she may preferably wear the stomach belt at all times, or periodically with its wear always in mind. Generally, patients who receive home oxygen therapy are expected to periodically use the stomach belt for at least several hours each day. As long as a respiratory rate measurement device is built into or attached to an oxygen concentration device, a change in pathological condition during the use of the oxygen concentration device can be checked by the respiratory rate measurement device reliably, and without forcing the patient to bear an additional burden. The respiratory rate measurement device is very useful.

As a method for obtaining the respiratory rate of a patient, which can be implemented in an oxygen concentration device, a method is available for mounting a micro-differential pressure sensor for respiratory measurement between the oxygen concentration device and a cannula fitted to the patient to measure a patient respiratory pressure during oxygen inhalation, and recording the patient respiratory pressure on a recording medium or transmitting it as communication data, as disclosed in PTL 1.

PTLs 2 and 4 disclose the fact that the respiratory rate and the like can be calculated from a respiratory pattern, but they describe no specific methods.

PTL 3 discloses a method for storing the timings at which the pressure waveform changes from a fall to a rise and counting the respiratory rate, based on the interval between these timings, and a method for multiplying the amplitude of the pressure by a predetermined detection level ratio and determining that breathing is present only when a threshold corresponding to the calculated product is exceeded.

PTL 5 discloses a method for calculating the respiratory rate by FFT (Fast Fourier Transform) processing or TDS processing.

PTL 6 discloses a method for measuring and storing pressure variations of an oxygen concentration device itself in advance, and subtracting the pressure variations from a detected pressure waveform.

PTL 7 discloses an oxygen concentrator taking advantage of pressure swing adsorption. A flow rate sensor is used to determine the flow rate of gas flowing through the outlet system to the user. A pressure sensor is used to monitor the pressure of the gas passing through conduit to the user. A controller collects and stores the number of breaths. Based on the number of breaths, or the average time between breaths, over the period of time a breathing rate may be calculated.

PTL 8 discloses analysing a photoplethysmograph (PPG) signal using autocorrelation sequence. e. The time between peaks of the combined autocorrelation sequence that are representative of the respiration information may be equivalent to the period of the respiration, which may be utilized to determine respiration rate (e.g., the frequency of respiration).

[CITATION LIST]

[PATENT LITERATURE]

[PTL 1] JP H6-190045 A
[PTL 2] JP 2001-286566 A
[PTL 3] JP H7-96035 A
[PTL 4] JP 2015-85191 A
[PTL 5] JP 2011-518016 A
[PTL 6] WO 2018-180392 A1
[PTL 7] US 2016/375215 A1
[PTL 8] US 2015/230759 A1

SUMMARY

In any method for directly sending the calculated respiratory data, since waveform data measured every 100 milliseconds (ms), for example, is directly transmitted, the amount of data is enormous, and it may take a long time for analysis.

The method for directly sending the calculated respiratory data has a drawback of, when an irregularity occurs in the respiratory waveform due to, e.g., noise or body movement, detecting a peak value or the like generated by the irregularity as breathing. The respiratory waveform considerably changes depending on the pathological condition of a patient who uses the respiratory rate measurement device, or the state of the patient, such as an active state or a sleep state, and even greatly varies in each individual patient. It is difficult to set a threshold as a criterion for breathing under a uniform rule, and determine whether breathing is present.

In the FFT, an enormous amount of calculation is involved, and, furthermore, when the interval of data used for arithmetic operation is set short, no accurate respiratory period may be calculated, and when the interval of the data is set too long, a peak of the respiratory period can hardly be obtained due to the influence of a slight shift in respiratory interval for each breath. The TDS processing poses a problem related to the influence of noise or body movement, a change in breathing that depends on the state of the patient, and differences in respiratory state for each individual patient.

A respiratory rate measurement device has been invented to solve the above-described problems, and has as its exemplary object to make it possible to improve the measurement accuracy of the respiratory rate, with a PSA oxygen concentration device being used in combination.

This object is accomplished by the subject-matter of the independent claim. The dependent claims concern particular embodiments.

According to this embodiment, the respiratory rate measurement device can improve the measurement accuracy of the respiratory rate, without forcing the patient to bear an additional burden, with a PSA oxygen concentration device being used in combination.

The objects and effects of the present invention will be appreciated and obtained by means of the elements and combinations particularly pointed out in the appended claims. Both the foregoing general description and the following detailed description are exemplary and explanatory, and do not limit the present invention described in the scope of claims.

BRIEF DESCRIPTION OF DRAWINGS

Fig. 1 is a diagram illustrating an exemplary schematic configuration of a PSA oxygen concentration device.

Fig. 2 is a diagram illustrating an exemplary schematic configuration of a respiratory rate measurement device.

Fig. 3 is a block diagram illustrating an exemplary microcomputer unit.

Fig. 4 is a chart representing pressure data including patient respiratory information, under the conditions in which continuous flow is set at 5 LPM, and an extension tube is added by 20 m.

Fig. 5 is a chart representing PSA pressure data extracted by an arithmetic operation unit, under the conditions in which continuous flow is set at 5 LPM, and an extension tube is added by 20 m.

Fig. 6 is a chart representing patient respiratory information after subtraction processing, under the conditions in which continuous flow is set at 5 LPM, and an extension tube is added by 20 m.

Fig. 7 is a conceptual diagram depicting the principle of separating patient respiratory information and a PSA pressure from pressure data including the PSA pressure and pressure variations caused by patient breathing.

Fig. 8 is a graph representing the result of calculating an autocorrelation coefficient from the patient respiratory information after the subtraction processing.

Fig. 9 is a flowchart illustrating exemplary processing of extracting patient respiratory information data.

Fig. 10 is a flowchart illustrating exemplary processing of estimating the respiratory rate, based on the patient respiratory information data.

DESCRIPTION OF EMBODIMENTS

A respiratory rate measurement device according to one aspect of an embodiment or embodiments will be described below with reference to the drawings. However, it should be noted that the technical scope of the present disclosure is not limited to these embodiments and encompasses the invention described in the scope of claims. In the following description and drawings, the same reference numerals denote components having the same functional configurations, and a repetitive description thereof will not be given.

<PSA Oxygen Concentration Device>

The configuration of a PSA oxygen concentration device, implemented as an exemplary pressure swing adsorption oxygen concentration device, used in combination with a respiratory rate measurement device according to one aspect of an embodiment will be described below.

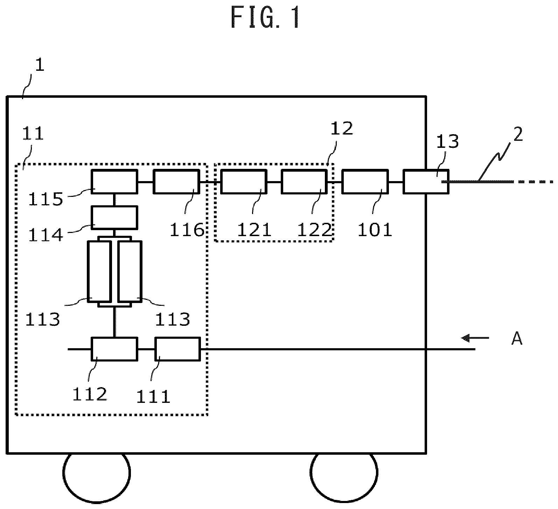

Fig. 1 is a diagram illustrating an exemplary schematic configuration of a PSA oxygen concentration device.

A PSA oxygen concentration device 1 includes an oxygen generation unit 11 that generates concentrated oxygen gas by introducing air A from the exterior of the PSA oxygen concentration device 1.

The air A introduced from the exterior of the PSA oxygen device into the oxygen generation unit 11 is compressed by a compressor 111 and fed into an adsorption column 113 via a first switching valve 112. The first switching valve 112 connects the compressor 111 to one of adsorption columns 113 to feed the compressed air into the connected adsorption column 113, and opens the remaining adsorption columns to the atmosphere.

The adsorption columns 113 are filled with adsorbents for selectively adsorbing nitrogen gas. The compressed air having passed through the adsorption column 113 reduces in nitrogen gas concentration and thus turns into concentrated oxygen gas. The concentrated oxygen gas is stored in a concentrated oxygen buffer tank 115 via a second switching valve 114. The second switching valve 114 connects or disconnects one of the adsorption columns 113 to or from the concentrated oxygen buffer tank 115.

The oxygen generation unit 11 uses the first switching valve 112 to connect the compressor 111 to one of the adsorption columns 113, and uses the second switching valve 114 to connect the adsorption column 113 connected to the compressor 111 to the concentrated oxygen buffer tank 115. Therefore, the compressor 111, one of the adsorption columns 113, and the concentrated oxygen buffer tank 115 are connected to each other, and generated concentrated oxygen gas is supplied to the concentrated oxygen buffer tank 115. The adsorption columns 113 that are not connected to the compressor 111, however, are opened to the atmosphere via the first switching valve 112, as they are disconnected from the concentrated oxygen buffer tank 115 by the second switching valve 114. With this operation, the adsorption columns 113 are depressurized to exhaust the nitrogen gas adsorbed to the adsorbents out of the PSA oxygen concentration device 1.

Opening and closing of the first switching valve 112 and the second switching valve 114 are controlled by, e.g., a microcomputer unit in a respiratory rate measurement device (not illustrated). The microcomputer unit can acquire a timing to switch between pressurization and depressurization in the adsorption columns 113. The respiratory rate measurement device may be placed in the oxygen concentration device 1, or may be placed outside the oxygen concentration device 1 separately from the oxygen concentration device 1.

As another example, the oxygen concentration device 1 may include an oxygen concentration control unit that controls oxygen concentration processing including processing of opening and closing the first and second switching valves. The microcomputer unit can acquire a timing to switch between pressurization and depressurization in the adsorption columns 113 from the oxygen concentration control unit.

The PSA oxygen concentration device 1 may connect two or more adsorption columns of the adsorption columns 113, in addition to the above-mentioned basic configuration. The PSA oxygen concentration device 1 may include an additional process such as a pressure equalization process of equalizing the pressures in the respective adsorption columns, or a purge process of refluxing part of the generated concentrated oxygen gas to one of the adsorption columns 113.

Normally, after the compressed air is fed into the adsorption column 113, the adsorption column 113 is opened to the atmosphere as it is disconnected from the compressor 111 by the first switching valve 112. In contrast to this, any adsorption column 113 opened to the atmosphere is connected to the compressor 111 by the first switching valve 112, and shifts to the process of oxygen compression. In this manner, the use of the first switching valve 112 to allow the adsorption columns 113 to alternately repeat compression and opening to the atmosphere makes it possible to continuously supply concentrated oxygen gas.

Since the pressure change in the adsorption column 113 upon concentrated oxygen generation is very large, periodical pressure variations occur in the pressure within an oxygen gas flow path, formed downstream of the adsorption column 113, upon switching of the adsorption column 113. The concentrated oxygen gas stored in the concentrated oxygen buffer tank 115 is regulated to attenuate the pressure variations by a pressure regulating valve 116.

The concentrated oxygen gas having its pressure regulated by the oxygen generation unit 11 has its oxygen flow rate controlled by an oxygen flow control unit 12 formed by a control valve 121 and a flowmeter 122, and is supplied to the exterior of the oxygen concentration device by an oxygen supply port 13 via a humidifier 101. In the oxygen flow control unit 12, either the control valve 121 or the flowmeter 122 may be provided upstream in the flow path, and the oxygen flow control unit 12 may include other configurations or arrangements.

The PSA oxygen concentration device 1 may include a switching fixed orifice for switching the flow rate, in place of the flowmeter 122 and the control valve 121. The PSA oxygen concentration device 1 may also use a scheme of manually controlling the flow rate using a visually-observable flowmeter such as a rotor meter as the flowmeter 122, and a manual flow control valve in place of the control valve 121, or may use other flow control schemes. The PSA oxygen concentration device 1 can even have a configuration equipped with no humidifier 101.

<Respiratory Rate Measurement Device>

An exemplary schematic configuration of a respiratory rate measurement device according to the present invention will be described below.

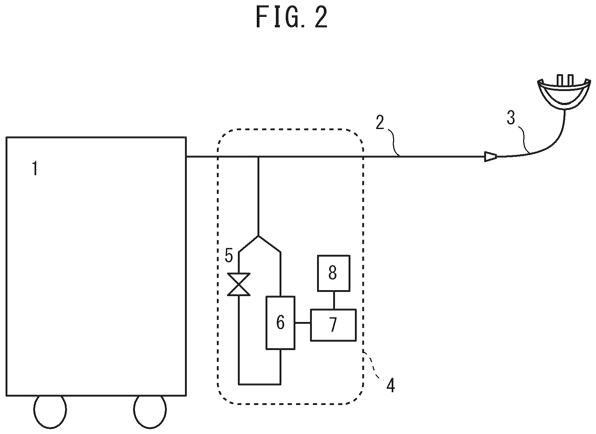

Fig. 2 is a diagram illustrating an exemplary schematic configuration of a respiratory rate measurement device.

Oxygen generated by the PSA oxygen concentration device 1 is supplied to the nasal cavity of a patient via a tube 2 connected to the PSA oxygen concentration device 1, and a nasal cannula 3 connected to the tube 2. The patient constantly breathes even during oxygen inhalation, and a pressure change occurring upon the breathing of the patient propagates toward the nasal cannula 3, the tube 2, and the PSA oxygen concentration device 1.

In this embodiment, to obtain the respiratory pressure of the patient, a respiratory rate measurement device 4 is connected to the tube 2 serving as an oxygen supply path. The tube refers to one including the entire tube provided between the humidifier 101 (the oxygen flow control unit 12 when the PSA oxygen concentration device 1 is equipped with no humidifier 101) and the nasal cannula 3, and the respiratory rate measurement device 4 may be connected anywhere in the tube.

A part or the whole of the respiratory rate measurement device 4 may be placed either inside or outside the PSA oxygen concentration device 1.

The inventors of the present invention made a close study, and discovered that even after the pressure variations are attenuated by the pressure regulating valve 116, pressure variations applied to the respiratory rate measurement device 4 include those occurring upon pressurization and depressurization during concentrated oxygen gas generation. Since the amplitude of the pressure variations occurring upon concentrated oxygen gas generation is larger than that of the respiratory pressure, and the amplitude of the respiratory pressure also reduces due to a pressure loss produced upon passage through the oxygen flow path, it is difficult to directly measure the respiratory pressure waveform of the patient from the pressure measured by the respiratory rate measurement device 4.

In this embodiment, the respiratory rate measurement device 4 includes a microcomputer unit 7 including an arithmetic operation unit and an estimation unit and connected to a pressure sensor 6. The pressure sensor 6 is preferably implemented as a micro-differential pressure sensor. Further, the respiratory rate measurement device 4 preferably includes a display unit 8, such as a liquid crystal display, that displays the measured respiratory rate. The display unit 8 is connected to the microcomputer unit 7 and controlled by the microcomputer unit 7.

In this embodiment, the respiratory rate measurement device 4 includes, e.g., a pressure sensor 6, and preferably a micro-differential pressure sensor 6, as a detection unit that detects and outputs the pressure in the tube, and a microcomputer unit 7 electrically connected to the detection unit. The respiratory rate measurement device 4 may further include a positive-displacement unit connected to the micro-differential pressure sensor 6, and a pressure smoothing unit formed by an orifice 5 connecting the tube and the positive-displacement unit to each other. The reason why the pressure smoothing unit is provided is as follows.

Since the respiratory pressure of the patient is normally about $\pm 10$ to 100 Pa, a sensor having the range of about $\pm$ 100 Pa is preferably used as the micro-differential pressure sensor 6 to obtain the respiratory pressure by the respiratory rate measurement device 4. In the state in which oxygen is supplied from the oxygen concentration device, a supply pressure is constantly generated upon oxygen supply, and the supply pressure generated upon oxygen supply exists at, e.g., about 300 Pa even for 1 LPM (litter per minute: l/min). This means that when one end of the micro-differential pressure sensor 6 is connected to the oxygen supply path, as described above, with the other end of the micro-differential pressure sensor being opened to the atmosphere, the pressure to be measured falls outside the measurement range of the micro-differential pressure sensor 6. Therefore, a pressure including respiratory information of the patient is preferably obtained in the measurement range of the micro-differential pressure sensor 6 by applying the pressure after passage through the orifice 5 to the other end of the micro-differential pressure sensor 6.

A pressure that falls within the measurement range of the micro-differential pressure sensor 6 may be preferably applied to the other end of the micro-differential pressure sensor 6, and a method for this operation is not limited to the example in this embodiment. As long as the measurement range of the micro-differential pressure sensor 6 is higher than the supply pressure generated upon oxygen supply, and a resolution high enough to allow detection of pressure variations occurring upon breathing of the patient can be ensured, the other end of the micro-differential pressure sensor 6 may even be opened to the atmosphere. A pressure sensor that measures a gauge pressure or an absolute pressure may be substituted for the micro-differential pressure sensor 6.

Since a pressure change upon breathing also appears as minute flow rate variations of the concentrated oxygen gas flowing through the tube, the respiratory rate measurement device 4 may use a flow sensor in place of the pressure sensor 6. The respiratory rate measurement device 4 may even include both the pressure sensor 6 and the flow sensor. The pressure sensor 6 and/or the flow sensor serves as a detection unit that detects an in-tube pressure and/or an in-tube gas flow rate in the tube 2 including respiratory information of the patient, and outputs pressure data and/or in-tube gas flow rate data.

<Microcomputer Unit>

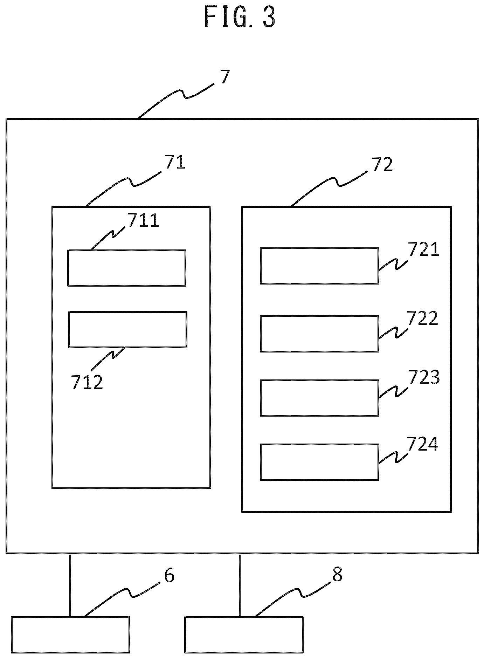

Fig. 3 is a diagram illustrating an exemplary configuration block of the microcomputer unit 7.

The microcomputer unit 7 including an arithmetic operation unit 722 and an estimation unit 723 is connected to the pressure sensor 6, and preferably the micro-differential pressure sensor 6. The arithmetic operation unit 722 and the estimation unit 723 obtain respiratory information of the patient by receiving the in-tube pressure data and or the in-tube gas flow rate data in the tube including the respiratory information of the patient, detected by the micro-differential pressure sensor 6 serving as the detection unit, and performing processing involved (to be described later).

The microcomputer unit 7 may be implemented as the same microcomputer as in a processing unit that processes, e.g., an oxygen generation function and a display and user interface function for the oxygen concentration device, or may be separate from the processing unit. When the microcomputer unit 7 is separate from the processing unit, it acquires a timing to switch a PSA period T from a microcomputer that processes the oxygen generation function, and uses the acquired timing for arithmetic operation.

The microcomputer unit 7 includes a storage unit 71 and a processing unit 72. The storage unit 71 is implemented as one or more semiconductor memories. The storage unit 71 includes at least one of nonvolatile memories such as a RAM, a flash memory, an EPROM, and an EEPROM. The storage unit 71 stores, e.g., a driver program, an operating system program, an application program, and data used for processing by the processing unit 72.

The storage unit 71 stores, as the driver program, e.g., a device driver program for controlling, e.g., the micro-differential pressure sensor 6 serving as the detection unit. A computer program may be installed in the storage unit 71 using, e.g., a known setup program from a computer-readable portable recording medium such as a CD-ROM or a DVD-ROM. The computer program may even be downloaded from, e.g., a program server and installed.

The storage unit 71 may further temporarily store temporary data associated with predetermined processing. The storage unit 71 stores, e.g., a threshold 711 and a variation value data file 712 for use in estimation of the respiratory rate.

The processing unit 72 includes one or more processors and their peripheral circuits. The processing unit 72 systematically controls the overall operation of the respiratory rate measurement device 4, and is implemented as, e.g.,

an MCU (Micro Control Unit).

The processing unit 72 performs processing based on the programs (e.g., the operating system program, the driver program, and the application program) stored in the storage unit 71. The processing unit 72 may execute several programs (e.g., the application program) in parallel. The processing unit 72 includes, e.g., a detected data acquisition unit 721, the arithmetic operation unit 722, the estimation unit 723, and a respiratory rate output unit 724.

Each of these units constituting the processing unit 72 may be implemented in the microcomputer unit 7 as an independent integrated circuit, circuit module, microprocessor, or firmware.

<Principle of Arithmetic Processing Performed in Embodiment>

The principle of processing performed in this embodiment, using data of respiratory information obtained by applying a respiratory pressure in a patient respiratory model from the nasal cannula 3 using the configuration illustrated in Fig. 2, will be described below.

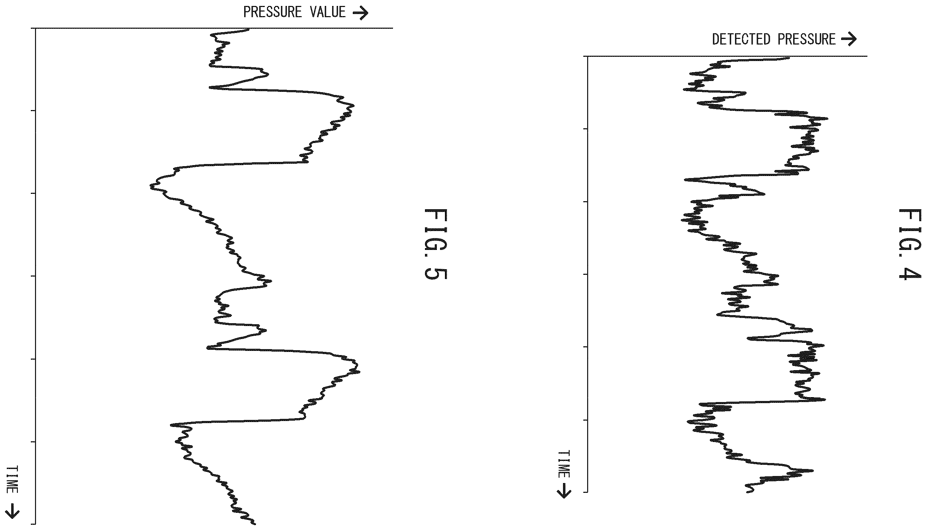

Figs. 4, 5, and 6 illustrate a group of data obtained when continuous flow is set at 5 LPM, and a 20-m extension tube is connected on the downstream side of the respiratory rate measurement device 4. The continuous flow means one concentrated oxygen gas supply scheme, in which concentrated oxygen gas is continuously supplied at a constant flow rate.

Fig. 4 is a chart representing pressure data including respiratory information of the patient and PSA pressure data of the PSA oxygen concentration device 1, when continuous flow is set at 5 LPM, and a 20-m extension tube is connected.

Fig. 5 is a chart representing PSA pressure data of the PSA oxygen concentration device 1 if the patient is not breathing or after respiratory components are removed, when continuous flow is set at 5 LPM, and a 20-m extension tube is connected.

The PSA pressure obtained by the PSA oxygen concentration device means a periodical pressure change related to the adsorption column period of the PSA oxygen concentration device 1 and occurring when oxygen is generated by the PSA oxygen concentration device 1, as described above. The period of the waveform of the PSA pressure coincides with the adsorption column switching period of the PSA oxygen concentration device 1.

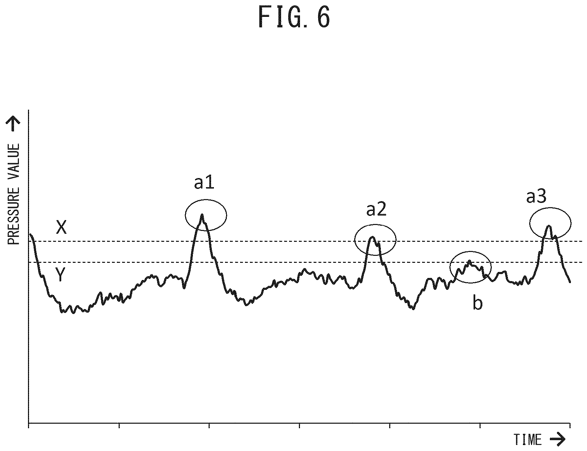

Fig. 6 is a chart representing the result of subtraction processing of components illustrated in Fig. 5 from components illustrated in Fig. 4 by software. The subtraction processing means processing of computing the difference between two pieces of data at an arbitrary time instant. By the subtraction processing, the respiratory rate measurement device 4 can remove PSA pressure components and detect a respiratory pressure in a patient respiratory model. By performing the subtraction processing by software, the respiratory rate measurement device 4 can obtain respiratory information of the patient even in the state in which oxygen is continuously inhaled at 5 LPM from the PSA oxygen concentration device 1 via the nasal cannula and the 20-m extension tube.

As a method for extracting patient respiratory information data by removing variation value data such as the PSA pressure components, the method for measuring and storing pressure variations of an oxygen concentration device itself in advance, and subtracting the pressure variations from a detected pressure waveform, as disclosed in PTL 6, may be used, or a method for subtracting pressure variations of an oxygen concentration device itself measured in real time from a detected pressure waveform may be used.

When the PSA oxygen concentration device switches between pressurization and depressurization with a single period T during at least a part of the total operation time, the arithmetic operation unit 722 extracts respiratory information by calculating, as the pressure at a certain time t, the difference between a flow rate value $Y(t)$ and a value $X(t)$ obtained by averaging $Y(t)$ and $Y(t - T)$, $Y(t - 2T)$,..., $Y(t - nT)$ (n is a preset arbitrary integer). An original respiratory waveform having the PSA pressure components removed can thus be accurately reproduced.

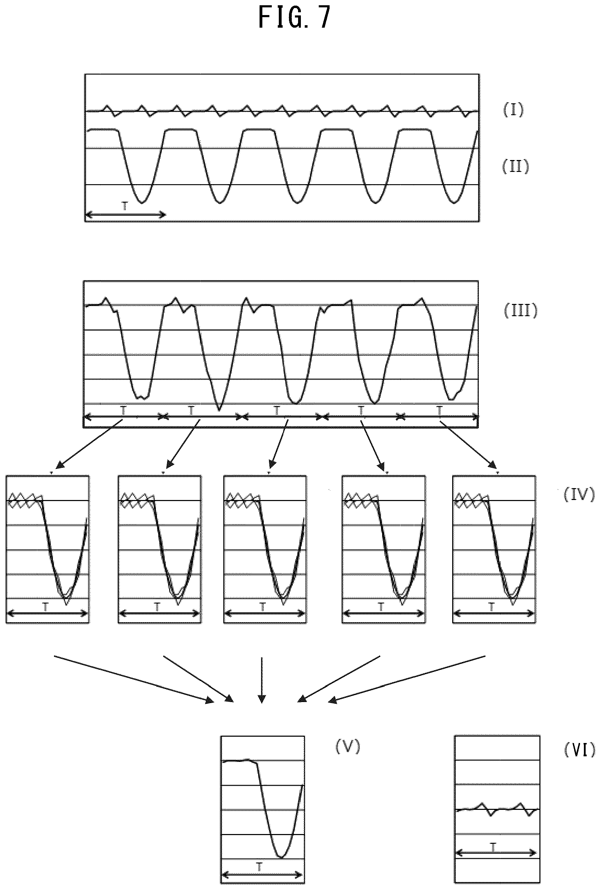

Fig. 7 is a conceptual diagram depicting arithmetic processing according to this embodiment. (I) illustrates a respiratory waveform, and (II) illustrates a model waveform for the PSA waveform. T denotes the period of the PSA waveform, which coincides with the switching period of the adsorption columns. (III) illustrates the sum of the waveforms illustrated in (I) and (II), and corresponds to the pressure measured in the tube portion. (IV) illustrates the result of dividing the waveform of (III) for each period T and superimposing the divided waveforms on each other. Unless the respiratory period and the PSA period completely coincide with each other, respiratory waveforms randomly appear in the waveforms divided by the periods T. (V) illustrates the result of averaging the waveforms superimposed on each other in (IV). Based on the measured pressure data and/or gas flow rate data (III), variation value data $X(t)$ representing a periodical pressure change and/or flow rate change of concentrated oxygen gas generated by the operation of the PSA oxygen concentration device is estimated by superimposition and averaging (V).

In one example, assuming T as one period, a simple moving average of five periods is used. (VI) illustrates a waveform obtained by subtracting the waveform of (V) from the portion corresponding to the last period T of the waveform of (III). This reveals that the original respiratory waveform (I) can be accurately reproduced. As an exemplary period T, the time from the start of the state in which one of the adsorption columns is connected to the compressor until a shift is made to the state in which another adsorption column is connected to the compressor may be selected. As another exemplary period T, the time from the start of the state in which one adsorption column is connected to the compressor, and then through the state in which the remaining adsorption columns are connected to the compressor, until a shift is made to the state in which the first adsorption column is connected to the compressor again may be selected. As still another example, a multiple of each switching time may be selected as T.

In this embodiment, in computing the waveform of (V) representing variation value data X(t) obtained by superimposition and averaging and estimated to bear the information of a periodical pressure change and/or flow rate change of the concentrated oxygen gas, a moving average of five periods is used, but another method may be used for averaging. The average value X(t) computed in the step of (V) is generally given by the following equation:

[Math. 1]

$$X(t) = \frac{\sum_{i=0}^{n} a_i Y(t-iT)}{\sum_{i=0}^{n} a_i} \quad \cdots \quad (1)$$

Throughout the entire description of the present disclosure, i does not represent an imaginary number, but it simply represents an integer variable i.

X(t) is the value after averaging processing at time t, and Y(t) is the actual measured value of the pressure at time t. $a_i$ is a weighting coefficient in weighted averaging, which can be obtained by selecting an arbitrary real number, n is the number of values of Y to be averaged. For, e.g., n = 4, and $a_0$ to $a_4$ = 1, the same computation as in Fig. 6 is performed. For $a_i = e^{-bi}$ (b is an arbitrary positive real number), and n = ∞, exponential smoothing is applicable.

Since it is difficult to handle infinity in actual computation, an asymptotic value is obtained for X(t) by sequential computation as, e.g., X(t) = (1 - $e^{-b}$)Y(t) + $e^{-b}$X(t - T). Appropriately selecting n and $a_i$ also allows averaging processing that achieves more rapid convergence, such as an FIR (Finite Impulse Response) filter.

Equation (1) is apparently seen as an equation for simply obtaining the time average of Y(t) by numerical computation. In normal time averaging, a value sufficiently smaller than the variation period of Y(t) is selected as T, while in this embodiment, it is important to select, as T, a value based on the adsorption and desorption period of a PSA process. This means, instead of simply temporally averaging the measured values Y(t), using the waveform of Y(t) over the period T as one unit to calculate the average of waveforms obtained for a duration of an integer multiple of T preceding a certain time of reference. Calculating the average of the waveforms makes it possible to accurately estimate a PSA pressure having characteristics periodically appearing at an interval equal to T.

In this example, a method for measuring the pressure has been exemplified as the respiratory rate measurement unit, but since the rate of flow through the tube also changes in response to the pressure variations in practice, a method for measuring the flow rate in place of the pressure can also be used. A method for measuring a pressure value and a flow rate value in combination or selectively, in accordance with the device operation conditions or environmental conditions, can even be used.

The respiratory information obtained by arithmetic processing is so-called raw data, which represents real-time information of breathing such as the pressure or the flow rate in the form of a waveform. This data may be directly recorded or transmitted, but in this case, the amount of data is enormous, and it takes a long time for analysis. It is, therefore, desired to automatically calculate respiratory rate data in the respiratory rate measurement device 4, and then record and/or transmit it.

As exemplified above, a detectable respiratory waveform contains a large number of noise components generated upon, e.g., airflow-related pressure variations or cannula oscillation. The respiratory period and the respiratory rate may not be appropriately calculated either by peak detection for calculating the timings at which the pressure changes from a decrease to an increase, or by a method for detecting the timings at which a value equal to or larger than a threshold has been reached.

Referring to, e.g., Fig. 6, respiratory waveforms to be counted appear as peaks indicated by a1, a2, and a3 in Fig. 6 and valleys appearing immediately after these peaks, and the remaining portions correspond to the noise components. Even when one attempts to detect these peaks as the timings at which the pressure changes from an increase to a decrease, it is highly probable that a portion indicated by b in Fig. 6 will be detected.

<Respiratory Rate Estimation Processing>

Even with a method for determining a portion having a value equal to or larger than a certain threshold as a peak, when the threshold is set to X, a peak as low as that indicated by a2 may fail to be detected, and when the threshold is set to Y, the noise component indicated by b may be detected as a peak. In this example, a waveform corresponding to only about three breaths is illustrated, but in an actual waveform, the levels of peaks or noise components may vary more seriously than those illustrated herein, and it is, therefore, very difficult to reliably identify peaks and set a threshold that allows reliable noise removal.

The inventors of the present invention conducted a close examination, and found that the respiratory rate can be detected with high detection performance not by the above-mentioned method, but by calculating an autocorrelation coefficient between an original waveform and a waveform shifted from the original waveform by a time $\Delta t$, and calculating $\Delta t$, in which the autocorrelation coefficient takes a peak, while changing $\Delta t$. These inventors also found a respiratory rate estimation method for estimating the respiratory rate per predetermined time from patient respiratory information.

An autocorrelation coefficient R can be calculated as the following equation:

[Math. 2]

$$R(\Delta t) = \frac{1}{(n - \Delta t)\sigma^2} \sum_{j=1}^{n - \Delta t/t_0} \{f(t - jt_0) - \mu\}[f\{t - (jt_0 + \Delta t)\} - \mu]$$

where $\Delta t$ is the amount of shift in time, $t_0$ is the data acquisition interval, n is the number of data used for one computation operation of the autocorrelation coefficient, and f(t) is the value of patient respiratory information data at time t, which is acquired n times at the interval $t_0$ in computation, $\mu$ and $\sigma$ are the average and the standard deviation of f(t), but in actual computation, the average and the standard deviation of the n obtained values of f(t) may be used. The method for calculating the autocorrelation coefficient in this embodiment is merely an example, and other methods for calculating the autocorrelation coefficient may be used.

The autocorrelation coefficient R calculated in this embodiment is multiplied by a normalization coefficient expressed as:

[Math. 3]

$$\frac{1}{(n - \Delta t)\sigma^2}$$

to define its value in the range of -1.0 to +1.0.

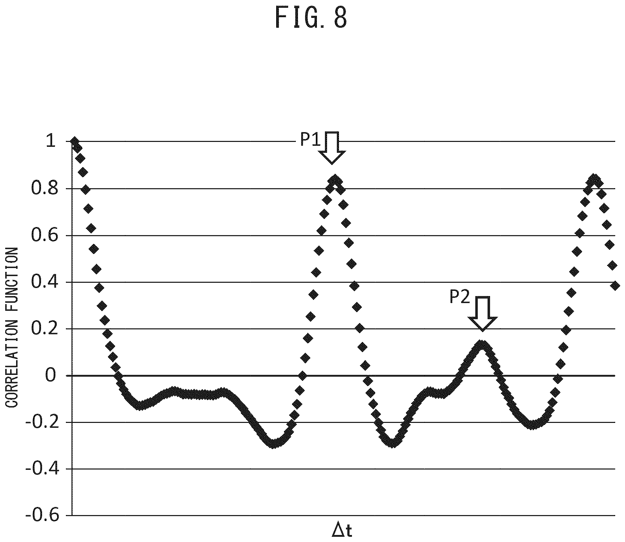

Fig. 8 is a graph generated by plotting, with respect to $\Delta t$, the computation result of the autocorrelation coefficient between the calculated waveform illustrated in Fig. 6 and the waveform obtained by shifting the original waveform along the time axis by the time $\Delta t$, using the above-mentioned equation multiplied by the normalization coefficient. The autocorrelation coefficient is computed every time $\Delta t$ is incremented by $t_0$ from $\Delta t = 0$. When $\Delta t = 0$, the autocorrelation coefficient is 1 because it represents the correlation between the original waveforms. After that, points P1 and P2 are obtained as points having a high correlation.

Fig. 8 reveals that even for a waveform that can hardly be used to determine the respiratory period either based on a threshold or by peak detection in raw patient respiratory information data, the respiratory period can easily be calculated by calculating the autocorrelation coefficient.

The data interval of f(t) used to compute the autocorrelation coefficient is determined from the range of the respiratory period to be calculated. The inventors of the present invention conducted a close examination, and discovered that the data interval of f(t) may be preferably set twice or more a minimum respiratory period to be calculated. The respiratory rate of an average adult is about 12 to 20 breaths per minute, which corresponds to a respiratory period of about 3 to 5 seconds. These inventors thus concluded that the data interval of f(t) may be preferably set to 5 seconds $\times$ 2 = 10 seconds.

It was found that the threshold of the autocorrelation coefficient for determining a peak of the autocorrelation coefficient may be preferably set to at least 0.3, and more preferably set in the range of 0.3 to 0.7. It was also found that setting the threshold below 0.3 increases the probability that an accidental rise in autocorrelation value due to, e.g., noise or baseline instability will be erroneously determined as a peak, and setting the threshold above 0.7 raises the probability that a rise in autocorrelation value corresponding to the respiratory period will be missed.

The respiratory rate is calculated as the reciprocal of the respiratory interval that is the value of $\Delta t$ when the

autocorrelation coefficient takes a certain value or more and a peak value. Δt at the point P1 that is the first peak in Fig. 8 is determined as the respiratory interval, and dividing one minute by the respiratory interval yields a respiratory rate per minute. The respiratory rate or the number of breaths per minute (bpm) is given by the following equation:

Respiratory Rate per Minute (bpm) = 60 Seconds/(Number of Steps at Peak Point × Sampling Time)

Depending on the respiratory waveform, a plurality of points may be present as peaks having values equal to or larger than a certain value, as illustrated in Fig. 8, but the right peak P2 is a peak corresponding to a multiple period appearing when Δt is shifted by several breaths. In estimating the respiratory rate, the peak P1 located leftmost, i.e., having the smallest value of Δt, which is estimated to correspond to the basic period of breathing, may be preferably used.

The computation described in this embodiment makes it possible to automatically accurately calculate respiratory rate information from the respiratory waveform obtained by the respiratory rate measurement unit connected to the concentrator.

When patient respiratory information data f(t) representing a change in information pertaining to, e.g., the pressure, based on exhalation and inhalation, contains only small noise other than breathing, the autocorrelation coefficient may accidentally become high with a certain period. In view of this, by additionally performing determination based on the variance of the patient respiratory information data f(t), the estimation unit 723 can achieve a higher accuracy by calculating no respiratory rate when no wave having a magnitude high enough to determine that breathing is expected to be included is available. The determination of the presence or absence of breathing is not limited to determination based on the variance of the patient respiratory information data f(t), and may be performed using other determination methods.

A variance $\sigma^2$ of the patient respiratory information data f(t) is calculated as the following equation:

[Math. 4]

$$\sigma^2 = \frac{1}{n}\sum_{t=1}^{n}(f(t) - \mu)^2$$

where n is the number of data used for one computation operation of the autocorrelation coefficient, f(t) is the value of patient respiratory information data at time t, and $\mu$ and $\sigma^2$ are the average and the variance of f(t).

As one example, the threshold for variance evaluation in the use of the PSA oxygen concentration device is set as follows:

When Respiratory Rate Is Less Than Eight Breaths: $\sigma^2 \geq 18$ (Pa$^2$)
When Respiratory Rate Is Eight to 10 Breaths: $\sigma^2 \geq 18$ (Pa$^2$)
When Respiratory Rate Is 11 Breaths or More: $\sigma^2 \geq 7$ (Pa$^2$)

The value of this threshold, however, may vary depending on the oxygen supply device used. Because of its variations that depend on parameters such as the respiratory rate, the threshold for variance evaluation may be appropriately set in accordance with the oxygen supply device used.

As one example, a first threshold $TH_{D1}$ for the variance $\sigma^2$ of the patient respiratory information data f(t) when the respiratory rate is less than eight breaths is set to $TH_{D1}$ = 18. A second threshold $TH_{D2}$ for the variance $\sigma^2$ of the patient respiratory information data f(t) when the respiratory rate is eight to 10 breaths is set to $TH_{D2}$ = 18. A third threshold $TH_{D3}$ for the variance $\sigma^2$ of the patient respiratory information data f(t) when the respiratory rate is 11 breaths or more is set to $TH_{D3}$ = 7. When the patient respiratory information data f(t) is equal to or higher than the threshold of the variance $\sigma^2$, the presence or absence of breathing is determined.

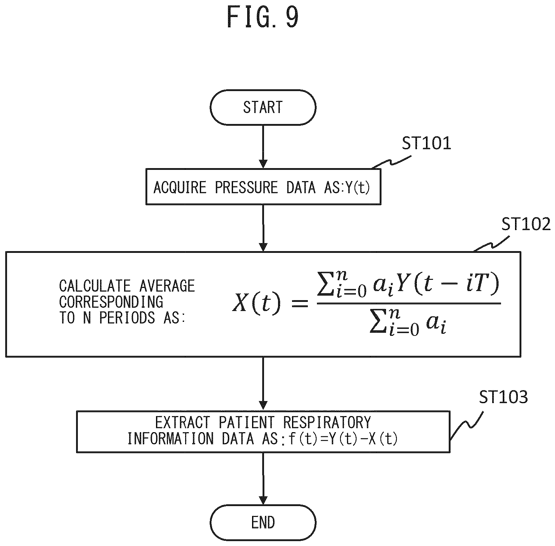

Fig. 9 is a flowchart illustrating exemplary processing of extracting patient respiratory information data.

The processing of extracting patient respiratory information data illustrated in Fig. 9 is performed by the microcomputer unit 7 in accordance with the computer program stored in the storage unit 71 in advance. The detected data acquisition unit 721 acquires pressure data Y(t) from the pressure sensor 6 serving as the detection unit (ST101). The arithmetic operation unit 722 calculates an average X(t) of Y(t) corresponding to n periods (ST102). The arithmetic operation unit 722 extracts patient respiratory information data f(t) by calculating the difference between Y(t) and X(t) (ST103). When PSA pressure variations measured and stored in advance are used, the arithmetic operation unit 722 does not perform the process of ST102, and extracts patient respiratory information data f(t) by calculating the difference between Y(t) and the PSA pressure variations measured and stored in advance, as ST103, after the process of ST101.

During the operation of the respiratory rate measurement device 4, processing of estimating the respiratory rate is repeatedly performed, so that patient respiratory information data f(t) is extracted and updated every time, for example, n pieces of measurement data of the pressures Y(t) are acquired at the interval $t_0$. The patient respiratory information data f(t) may even be extracted at a predetermined interval and be updated.

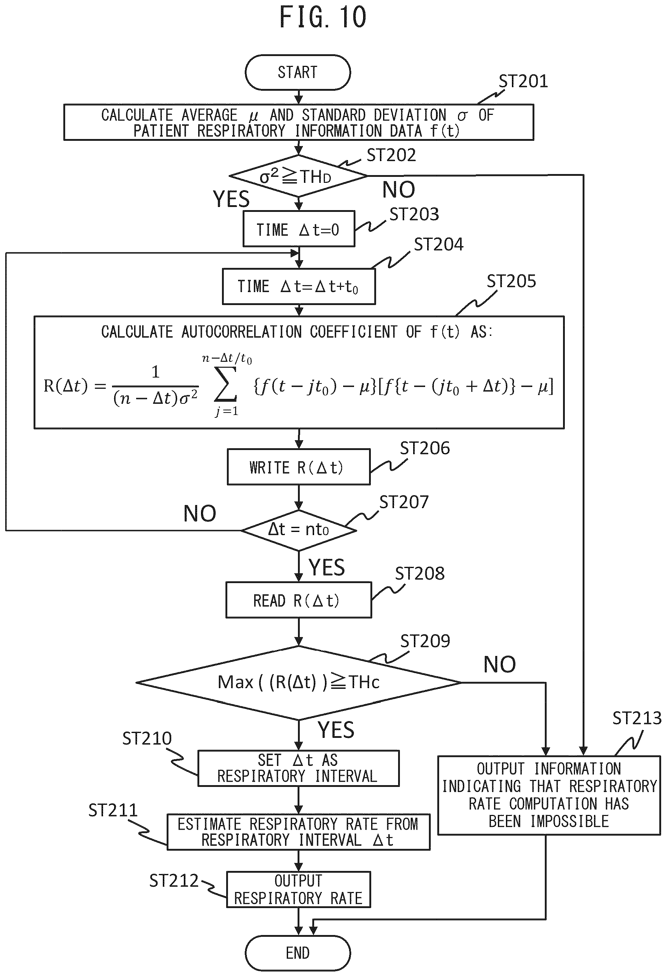

Fig. 10 is a flowchart illustrating exemplary processing of estimating the respiratory rate, based on the patient respiratory information data.

The processing of estimating the respiratory rate illustrated in Fig. 10 is performed by the microcomputer unit 7 in accordance with the computer program stored in the storage unit 71 in advance. The estimation unit 723 calculates an average $\mu$ and a standard deviation $\sigma$ of the patient respiratory information data f(t) (ST201). First, to determine the presence or absence of a respiratory rate, the estimation unit 723 determines whether the variance $\sigma^2$ that is the square of the standard deviation $\sigma$ is equal to or higher than a predetermined threshold $TH_D$ (ST202). When the variance $\sigma^2$ is lower than the threshold $TH_D$ (NO in ST202), the respiratory rate output unit 724 outputs information indicating that computation has been impossible (ST213), and ends the process. When the variance $\sigma^2$ is equal to or higher than the threshold $TH_D$ (YES in ST202), the estimation unit 723 sets the time $\Delta t$ to zero (0) as the amount of shift in time (ST203).

The estimation unit 723 increments the time $\Delta t$ by the data acquisition interval $t_0$ (ST204). The estimation unit 723 calculates an autocorrelation coefficient $R(\Delta t)$ of the patient respiratory information data f(t) for the time $\Delta t$ (ST205). The estimation unit 723 writes the calculated autocorrelation coefficient $R(\Delta t)$ into the storage unit 71 (ST206). The estimation unit 723 determines whether the time $\Delta t$ has reached $nt_0$ (ST207). When the time $\Delta t$ has not reached $nt_0$, the process returns to ST203, in which estimation unit 723 repeats a series of processes (NO in ST207).

When the time $\Delta t$ has reached $nt_0$ (YES in ST207), the estimation unit 723 reads the autocorrelation coefficients $R(\Delta t)$ stored in the storage unit 71 (ST208). The estimation unit 723 compares the read autocorrelation coefficients $R(\Delta t)$ with each other to determine whether a maximum autocorrelation coefficient $Max(R(\Delta t))$, i.e., a peak autocorrelation coefficient $R(\Delta t)$ is present (ST209). More specifically, the estimation unit 723 determines whether $Max(R(\Delta t))$ is equal to or higher than a predetermined threshold THc.

When the maximum autocorrelation coefficient $Max(R(\Delta t))$ is equal to or higher than the predetermined threshold THe (YES in ST209), the estimation unit 723 sets the time $\Delta t$ for the maximum autocorrelation coefficient $Max(R(\Delta t))$ as a respiratory interval (ST210). The estimation unit 723 estimates the respiratory rate from the respiratory interval $\Delta t$ (ST211). The respiratory rate output unit 724 outputs a respiratory rate signal (ST212), and ends the process. For example, the display unit 8, upon receiving the respiratory rate signal, displays the respiratory rate.

When the maximum autocorrelation coefficient $Max(R(\Delta t))$ is lower than the predetermined threshold THe (NO in ST209), the respiratory rate output unit 724 outputs information indicating that computation has been impossible (ST213), and ends the process. During the operation of the respiratory rate measurement device 4, processing of estimating the respiratory rate is repeatedly performed, so that the respiratory rate measurement device 4 can update the respiratory rate and display it on the display unit 8. The display on the display unit 8 may be performed at a predetermined interval.

REFERENCE SIGNS LIST

    1 PSA oxygen concentration device
    2 Tube
    3 Nasal cannula
    4 Respiratory rate measurement device
    5 Orifice
    6 Pressure sensor
    7 Microcomputer unit
    71 Storage unit
    72 Processing unit
    721 Detected data acquisition unit
    722 Arithmetic operation unit
    723 Estimation unit
    724 Respiratory rate output unit
    8 Display unit

**Claims**

1. A respiratory rate measurement device (4) comprising:

a detection unit (6) adapted to detect an in-tube pressure and/or an in-tube gas flow rate in a tube (2) supplying concentrated oxygen gas to a patient from a pressure swing adsorption oxygen concentration device (1) connected to the patient and concentrating oxygen in the air by periodically repeating pressurization and depressurization, and output pressure data and/or gas flow rate data;
an arithmetic operation unit (722) adapted to extract patient respiratory information data, based on the pressure data and/or the gas flow rate data; and
an estimation unit (723) adapted to estimate a respiratory rate per predetermined time, based on the patient respiratory information data,
**characterised in that**
the arithmetic operation unit (722) is adapted to estimate variation value data representing a periodical pressure change and/or flow rate change of the concentrated oxygen gas occurring upon an operation of an oxygen concentration device, based on the pressure data and/or the gas flow rate data, and extract the patient respiratory information data by removing the variation value data from the pressure data and/or the gas flow rate data, and
**in that**
the estimation unit (723) is adapted to calculate an autocorrelation coefficient between the patient respiratory information data and data shifted from the patient respiratory information data by a time $\Delta t$, while changing the time $\Delta t$, and estimate the respiratory rate using, as a respiratory interval, a time $\Delta t$ in which the autocorrelation coefficient takes a peak.

2. The respiratory rate measurement device (4) according to claim 1, wherein the estimation unit (723) is adapted to estimate the respiratory rate, using a waveform indicating the patient respiratory information data of at least 10 seconds.

3. The respiratory rate measurement device according to claim 1 or 2, wherein the estimation unit (723) is adapted to use a peak of the autocorrelation coefficient exceeding a predetermined threshold to estimate the respiratory rate.

4. The respiratory rate measurement device according to claim 3, wherein the threshold takes a value of 0.3 to 0.7.

5. The respiratory rate measurement device (4) according to claim 3 or 4, wherein information indicating that computation has been impossible is output when a peak of the autocorrelation coefficient exceeding the predetermined threshold fails to be obtained.

6. The respiratory rate measurement device (4) according to any one of claims 1 to 5, further comprising a storage unit (71) adapted to store the variation value data measured in advance,
wherein the arithmetic operation unit (722) is adapted to extract the patient respiratory information data by removing the variation value data from the pressure data and/or the gas flow rate data.

**Patentansprüche**

1. Atemfrequenzmessvorrichtung (4), umfassend:

eine Detektionseinheit (6), die dazu ausgelegt ist, einen Druck im Schlauch und/oder eine Gasdurchflussrate im Schlauch in einem Schlauch (2) zu erfassen, der einem Patienten konzentriertes Sauerstoffgas von einer Druckwechseladsorptions-Sauerstoffkonzentrationsvorrichtung (1) zuführt, die mit dem Patienten verbunden ist und Sauerstoff in der Luft durch periodische Wiederholung der Druckbeaufschlagung und Druckentlastung, und der Ausgabe von Druckdaten und/oder Gasdurchflussratendaten auszugeben;
eine arithmetische Betriebseinheit (722), die dazu ausgelegt ist, Patienten-Atmungsinformationsdaten basierend auf den Druckdaten und/oder den Gasdurchflussratendaten zu extrahieren; und
eine Schätzeinheit (723), die dazu ausgelegt ist, eine Atemfrequenz pro vorbestimmter Zeit basierend auf den Patienten-Atmungsinformationsdaten zu schätzen,
**dadurch gekennzeichnet, dass** die arithmetische Betriebseinheit (722) dazu ausgelegt ist, Variationswertdaten zu schätzen, die eine periodische Druckänderung und/oder Durchflussratenänderung des konzentrierten Sauerstoffgases darstellen, die bei einem Betrieb einer Sauerstoffkonzentrationsvorrichtung auftreten, basierend auf den Druckdaten und/oder den Gasdurchflussratendaten, und die Patienten-Atmungsinformationsdaten zu extrahieren, indem die Variationswertdaten aus den Druckdaten und/oder den Gasdurchflussratendaten entfernt werden, und dass

die Schätzeinheit (723) ist dazu ausgelegt, einen Autokorrelationskoeffizienten zwischen den Patienten-Atmungsinformationsdaten und Daten, die von den Patienten-Ateminformationsdaten um eine Zeit Δt verschoben sind, zu berechnen, während die Zeit Δt geändert wird, und die Atemfrequenz zu schätzen, indem als Atemintervall eine Zeit Δt verwendet wird, in der der Autokorrelationskoeffizient einen Spitzenwert annimmt.

2. Atemfrequenzmessvorrichtung (4) nach Anspruch 1, wobei die Schätzeinheit (723) dazu ausgelegt ist, die Atemfrequenz unter Verwendung einer Wellenform zu schätzen, die die Patienten-Ateminformationsdaten von mindestens 10 Sekunden anzeigt.

3. Atemfrequenzmessvorrichtung nach Anspruch 1 oder 2, wobei die Schätzeinheit (723) dazu ausgelegt ist, einen Peak des Autokorrelationskoeffizienten, der einen vorbestimmten Schwellenwert überschreitet, zur Schätzung der Atemfrequenz zu verwenden.

4. Atemfrequenzmessvorrichtung nach Anspruch 3, wobei der Schwellenwert einen Wert von 0,3 bis 0,7 annimmt.

5. Atemfrequenzmessvorrichtung (4) nach Anspruch 3 oder 4, wobei Information, die anzeigt, dass eine Berechnung unmöglich war, ausgegeben wird, wenn ein Peak des Autokorrelationskoeffizienten, der den vorbestimmten Schwellenwert überschreitet, nicht erreicht wird.

6. Atemfrequenzmessvorrichtung (4) nach einem der Ansprüche 1 bis 5, ferner umfassend eine Speichereinheit (71), die dazu ausgelegt ist, die im Voraus gemessenen Variationswertdaten zu speichern,
wobei die arithmetische Betriebseinheit (722) dazu ausgelegt ist, die Atmungsdaten des Patienten zu extrahieren, indem die Variationswertdaten aus den Druckdaten und/oder den Gasdurchflussratendaten entfernt werden.

## Revendications

1. Dispositif de mesure de la fréquence respiratoire (4) comprenant:

   une unité de détection (6) adaptée pour détecter une pression en tube et/ou un débit de gaz en tube dans un tube (2) fournissant de l'oxygène gazeux concentré à un patient à partir d'un dispositif de concentration d'oxygène par adsorption modulée en pression (1) connecté au patient et concentrant l'oxygène dans l'air en répétant périodiquement une pressurisation et une dépressurisation, et produire des données de pression et/ou des données de débit de gaz;
   une unité d'opération arithmétique (722) adaptée pour extraire des données d'informations respiratoires du patient sur la base des données de pression et/ou des données de débit de gaz; et
   une unité d'estimation (723) adaptée pour estimer une fréquence respiratoire pendant une durée prédéterminée, sur la base des données d'informations respiratoires du patient,
   **caractérisé en ce que**
   l'unité d'opération arithmétique (722) est adaptée pour estimer des données de valeur de variation représentant un changement de pression et/ou un changement de débit périodique de l'oxygène gazeux concentré se produisant lors d'un actionnement d'un dispositif de concentration d'oxygène, sur la base des données de pression et/ou des données de débit de gaz, et extraire les données d'informations respiratoires du patient en retirant les données de valeur de variation des données de pression et/ou des données de débit de gaz, et **en ce que** l'unité d'estimation (723) est adaptée pour calculer un coefficient d'autocorrélation entre les données d'informations respiratoires du patient et des données décalées des données d'informations respiratoires du patient d'un temps Δt, tout en modifiant le temps Δt, et estimer la fréquence respiratoire en utilisant, comme intervalle respiratoire, un temps Δt pendant lequel le coefficient d'autocorrélation atteint un pic.

2. Dispositif de mesure de la fréquence respiratoire (4) selon la revendication 1, dans lequel l'unité d'estimation (723) est adaptée pour estimer la fréquence respiratoire, en utilisant une forme d'onde indiquant les données d'informations respiratoires du patient d'au moins 10 secondes.

3. Dispositif de mesure de la fréquence respiratoire selon la revendication 1 ou 2, dans lequel l'unité d'estimation (723) est adaptée pour utiliser un pic du coefficient d'autocorrélation dépassant un seuil prédéterminé pour estimer la fréquence respiratoire.

4. Dispositif de mesure de la fréquence respiratoire selon la revendication 3, dans lequel le seuil prend une valeur de 0,3 à 0,7.

5. Dispositif de mesure de la fréquence respiratoire (4) selon la revendication 3 ou 4, dans lequel des informations indiquant que le calcul a été impossible sont délivrées lorsqu'un pic du coefficient d'autocorrélation dépassant le seuil prédéterminé ne parvient pas à être obtenu.

6. Dispositif de mesure de la fréquence respiratoire (4) selon l'une quelconque des revendications 1 à 5 comprenant en outre une unité de stockage (71) adaptée pour stocker les données de valeur de variation mesurées à l'avance, dans lequel l'unité d'opération arithmétique (722) est adaptée pour extraire les données d'informations respiratoires du patient en retirant les données de valeur de variation des données de pression et/ou des données de débit de gaz.

FIG. 1

FIG. 2

FIG. 3

## FIG. 4

## FIG. 5

# FIG. 6

# FIG. 7

# FIG. 8

# FIG. 9

START

ACQUIRE PRESSURE DATA AS:Y(t)　　　ST101

CALCULATE AVERAGE
CORRESPONDING
TO N PERIODS AS:　　　$X(t) = \dfrac{\sum_{i=0}^{n} a_i Y(t - iT)}{\sum_{i=0}^{n} a_i}$　　　ST102

EXTRACT PATIENT RESPIRATORY
INFORMATION DATA AS:f(t)=Y(t)-X(t)　　　ST103

END

# FIG. 10

START

CALCULATE AVERAGE $\mu$ AND STANDARD DEVIATION $\sigma$ OF PATIENT RESPIRATORY INFORMATION DATA f(t) — ST201

ST202 — $\sigma^2 \geqq TH_D$

YES / NO

TIME $\Delta t = 0$ — ST203

TIME $\Delta t = \Delta t + t_0$ — ST204

CALCULATE AUTOCORRELATION COEFFICIENT OF f(t) AS: — ST205

$$R(\Delta t) = \frac{1}{(n - \Delta t)\sigma^2} \sum_{j=1}^{n - \Delta t/t_0} \{f(t - jt_0) - \mu\}[f\{t - (jt_0 + \Delta t)\} - \mu]$$

WRITE $R(\Delta t)$ — ST206

NO — $\Delta t = nt_0$ — ST207

YES

READ $R(\Delta t)$ — ST208

ST209 — $Max((R(\Delta t)) \geqq TH_C$ — NO

YES

SET $\Delta t$ AS RESPIRATORY INTERVAL — ST210

OUTPUT INFORMATION INDICATING THAT RESPIRATORY RATE COMPUTATION HAS BEEN IMPOSSIBLE — ST213

ESTIMATE RESPIRATORY RATE FROM RESPIRATORY INTERVAL $\Delta t$ — ST211

OUTPUT RESPIRATORY RATE — ST212

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H6190045 A
- JP 2001286566 A
- JP H796035 A
- JP 2015085191 A
- JP 2011518016 A
- WO 2018180392 A1
- US 2016375215 A1
- US 2015230759 A1